# EUROPEAN PATENT SPECIFICATION

(11) **EP 3 603 697 B1**
(45) Date of publication and mention of the grant of the patent: **03.08.2022**
(21) Application number: 18778072.1
(22) Date of filing: 21.02.2018
(51) Int. Cl.: A61M 1/36

(54) **BLOOD PURIFICATION DEVICE**
BLUTREINIGUNGSVORRICHTUNG
DISPOSITIF DE PURIFICATION DE SANG

(30) Priority: 31.03.2017 JP 2017072424
(43) Date of publication of application: 05.02.2020
(73) Proprietor: Asahi Kasei Medical Co., Ltd., Tokyo 100-0006 (JP)
(72) Inventor: HINO, Mayumi, Kawagoe-shi Saitama 350-0833 (JP)
(74) Representative: dompatent von Kreisler Selting Werner - Partnerschaft von Patent- und Rechtsanwälten mbB
(86) International application number: PCT/JP2018/006244
(87) International publication number: WO 2018/180043

(56) References cited:
- JP-A- 2002 085 555
- JP-A- 2005 224 597
- US-A- 4 215 688
- US-A- 4 215 688
- US-A1- 2015 021 244

## Description

### Technical Field

The present invention relates to a blood purification device

### Background Art

Blood purification therapies have conventionally been performed by taking out the blood from a patient's body, removing a disease-causing substance, a specific leukocyte, or the like from this blood and reinfusing the resulting blood to the body. In such blood purification therapies, a blood purification device has been used which has a blood circuit for circulating the blood taken out from a body and reinfusing it thereto and a blood purifier equipped with a treating material such as adsorbing material or separating material for removing or separating a specific substance from the blood.

The blood circuit of such a blood purification device is connected to a patient's blood vessel via a puncture needle. Recently, there has been proposed an automatic blood return device for returning the blood which has remained in a blood removal line to a patient via a puncture needle by performing reverse rotation of a blood pump provided in a blood circuit (refer to, for example, Patent Document 1). US 2015/0021244 discloses a blood purification apparatus providing for an automatic blood return at the end of the blood purification process. US 4,215,688 discloses an apparatus for the extra corporeal treatment of diseases. The apparatus may comprise two transfusion lines, one with saline and the other with an anticoagulant each contained in a container.

### Citation List

### Patent Document

Patent Document 1: Japanese Patent Application Laid-Open No. 2005-224597 Summary

### Technical Problem

The blood circuit of a blood purification device has a blood removal line for delivering a blood taken out from a patient to a blood purifier. To this blood removal line, a transfusion line for supplying physiological saline or a transfusion line for supplying an anticoagulant is sometimes connected. When a transfusion line for supplying a transfusion such as anticoagulant is connected to a blood removal line and blood return is performed by the reverse rotation of a blood pump as usual, a transfusion supply unit for blood return should be provided on a side downstream of the blood pump. In a blood purification therapy not requiring a dialysis solution or the like, such a transfusion supply unit for blood reinfusion has been unnecessary.

With the foregoing in view, the present invention has been made. The purpose is to, in a blood purification device in which a transfusion line for supplying a transfusion such as an anticoagulant is connected to a blood removal line, return the blood automatically without performing reverse rotation of a blood pump and without providing a transfusion supply unit for blood return on a side downstream of the blood pump.

### Solution to Problem

In order to achieve the above-described purpose, a blood purification device according to the present invention is equipped with a blood removal line for delivering the blood taken out from a patient to a blood purifier, a blood pump placed on the blood removal line, a first transfusion line for supplying a first transfusion to the blood removal line on a side upstream of the blood pump, a transfusion vessel connected to the first transfusion line, a first opening/closing valve for opening or closing the first transfusion line, a second transfusion line for supplying a second transfusion to the blood removal line on a side upstream of the blood pump, a transfusion pump placed on the second transfusion line, a second opening/closing valve placed in the blood removal line on a side upstream of the first transfusion line and the second transfusion line for opening or closing the blood removal line, and a control unit for controlling the blood pump, the transfusion pump, the first opening/closing valve and the second opening/closing valve. In this device, the control unit achieves automatic return of the blood to the patient by successively performing the following steps of: a first step of stopping the operation of the blood pump and the transfusion pump, and opening the first transfusion line by means of the first opening/closing valve and closing the blood removal line by means of the second opening/closing valve, a second step of performing forward rotation of the blood pump by a predetermined volume X and thereby moving the first transfusion from the first transfusion line to the blood removal line, a third step of performing reverse rotation of the transfusion pump by a predetermined volume Y and thereby moving the first transfusion in the blood removal line to the second transfusion line, a fourth step of closing the first transfusion line by means of the first opening/closing valve and opening the blood removal line by the second opening/closing valve, and a fifth step of performing forward rotation of the transfusion pump by a predetermined volume Z to move the first transfusion in the second transfusion line to the blood removal line and return the first transfusion to the patient. In this device, as the first transfusion, physiological saline can be used. As the second transfusion, a liquid different from physiological saline (for example, an anticoagulant-containing liquid) can be used.

When such a constitution is used, the control unit can achieve automatic blood return to a patient by controlling the blood pump, the

In addition, since the forward rotation volume (X) of the blood pump, the reverse rotation volume (Y) of the transfusion pump, and the forward rotation volume (Z) of the transfusion pump are specified respectively, the amount of the blood to be returned to the patient can be controlled precisely.

In the blood purification device according to the present invention, the first transfusion line can be connected to the blood removal line on a side upstream of the second transfusion line. On the other hand, the first transfusion line may be connected to the blood removal line on a side downstream of the second transfusion line. The first transfusion line may also be connected to the second transfusion line on a side downstream of the transfusion pump.

In the blood purification device according to the present invention, a chamber may be provided in the second transfusion line on a side downstream of the transfusion pump.

In the blood purification device according to the present invention, the predetermined volume X can be set at an internal volume or more of a portion of the blood removal line positioned between the first transfusion line and the second transfusion line. The predetermined volume Y can be set at an internal volume or more of a portion of the blood removal line positioned between a patient-side end of the blood removal line and either one of the first transfusion line or the second transfusion line which is on an upstream side. The predetermined volume Z can be set at an internal volume or more of a portion of the blood removal line positioned between a patient-side end of the blood removal line and either one of the first transfusion line or the second transfusion line which is on an upstream side.

In the blood purification device according to the present invention, a first air bubble detector can be provided in the blood removal line on a side upstream of the first transfusion line and the second transfusion line. In such a case, in performing the fifth step, the control unit can stop the transfusion pump (and/or close the blood removal line by means of the second opening/closing valve) when the first air bubble detector detects air bubbles.

By using such a constitution, when the first transfusion in the second transfusion line is moved to the blood removal line and returned to the patient by performing forward rotation of the transfusion pump by the predetermined volume Z and the first air bubble detector provided on a side upstream of the first transfusion line and the second transfusion line detects air bubbles, the transfusion pump can be stopped (and/or the blood removal line can be closed). This makes it possible to prevent the transfusion or blood to be returned to the patient from containing air bubbles.

The blood purification device according to the present invention may be equipped with a blood return line for returning the blood purified by the blood purifier to the patient. In such a case, the control unit can perform, before the first step or after the fifth step, a sixth step of performing forward rotation of the blood pump by a predetermined volume W while opening the first transfusion line by means of the first opening/closing valve and closing the blood removal line by means of the second opening/closing valve. The predetermined volume W can be set at the sum or more of the internal volume of a portion of the blood removal line on a side downstream of the second transfusion line, the internal volume of the blood purifier, and the internal volume of the blood return line.

By using such a constitution, before the first step of stopping the operation of the blood pump and the transfusion pump or after the fifth step of performing forward rotation of the transfusion pump by a predetermined volume Z to move the first transfusion in the second transfusion line to the blood removal line and return the first transfusion to the patient, the sixth step of performing forward rotation of the blood pump by a predetermined volume W while opening the first transfusion line and closing the blood removal line can be performed. In short, before blood return is performed via the blood removal line or after the transfusion is returned sufficiently to the patient via the blood removal line, blood return can be performed via the blood return line.

In the blood purification device according to the present invention, a first air bubble detector can be provided in the blood removal line on a side upstream of the first transfusion line and the second transfusion line. In such a case, when the first air bubble detector detects air bubbles in the fifth step, the control unit can perform the sixth step.

By using such a constitution, when the first air bubble detector provided in the blood removal line detects air bubbles in the fifth step of performing forward rotation of the transfusion pump by a predetermined volume Z to move the first transfusion in the second transfusion line to the blood removal line and return the first transfusion to the patient, it is possible to proceed to the sixth step of performing forward rotation of the blood pump by a predetermined volume W while opening the first transfusion line and closing the blood removal line. It is therefore possible to prevent the transfusion to be returned to the patient from containing air bubbles and at the same time, to proceed to the sixth step without requiring an additional operation by an operator.

The blood purification device according to the present invention can be provided with a second air bubble detector in the blood return line. In such a case, the control unit can terminate the operation when the second air bubble detector detects air bubbles in the sixth step.

By using such a constitution, when the second air bubble detector provided in the blood return line detects air bubbles in the sixth step of performing forward rotation of the blood pump by a predetermined volume W while opening the first transfusion line and closing the blood removal line, the operation can be terminated. This makes it possible to prevent the blood returned to the patient via the blood return line from containing air bubbles.

The blood purification device according to the present invention can be provided with a first pressure sensor placed between the blood pump on the blood removal line and the patient for measuring the pressure in the blood removal line. In such a case, when the pressure measured by the first pressure sensor exceeds a predetermined value in the fifth step, the control unit can perform the sixth step. The first pressure sensor is only required to measure the pressure in the blood removal line and it may be placed on the first transfusion line or on the second transfusion line.

By using such a constitution, when the pressure measured by the first pressure sensor placed between the blood pump on the blood removal line and the patient exceeds a predetermined value in the fifth step of performing forward rotation of the transfusion pump by a predetermined volume Z to move the first transfusion in the second transfusion line to the blood removal line and return the first transfusion to the patient, it is possible to proceed to the sixth step of performing forward rotation of the blood pump by a predetermined volume W while opening the first transfusion line and closing the blood removal line. In short, it is possible to switch to blood return via the blood return line without forcibly pouring the transfusion when the connection part with the patient is clogged with a clot.

The blood purification device according to the present invention can be provided with a bypass line for bypassing the blood purifier, a third opening/closing valve for opening or closing the bypass line, and a second pressure sensor placed between the blood pump and the blood purifier. In such a case, when the pressure measured by the second pressure sensor exceeds a predetermined value in the sixth step, the control unit can open the bypass line by means of the third opening/closing valve. The third opening/closing valve may be a pump. In this case, when the pressure measured by the second pressure sensor exceeds a predetermined value in the sixth step, the control unit can perform blood return by this pump via the bypass line.

By using such a constitution, when the pressure measured by the second pressure sensor placed between the blood pump on the blood removal line and the blood purifier exceeds a predetermined value (for example, when coagulation occurs in the blood purifier) in the sixth step of performing forward rotation of the blood pump by a predetermined volume W while opening the first transfusion line and closing the blood removal line, the bypass line for bypassing the blood purifier can be opened.

The blood purification device according to the present invention can be provided with a third pressure sensor for measuring the pressure in the blood return line. In such a case, when the pressure measured by the third pressure sensor exceeds a predetermined value in the sixth step, the control unit can terminate the operation.

By using such a constitution, when the pressure measured by the third pressure sensor placed in the blood return line exceeds a predetermined value (for example, when the connection part with the patient is clogged with a clot and further blood return is prevented) in the sixth step of performing forward rotation of the blood pump by a predetermined volume W while opening the first transfusion line and closing the blood removal line, the operation can be terminated.

The blood purification device according to the present invention can be provided with a bypass line for bypassing the blood purifier and a third opening/closing valve for opening or closing the bypass line. In such a case, when a difference between the pressure measured by the second pressure sensor and the pressure measured by the third pressure sensor exceeds a predetermined value in the sixth step, the control unit can open the bypass line by means of the third opening/closing valve.

By using such a constitution, when a difference between the pressure measured by the second pressure sensor placed between the blood pump on the blood removal line and the blood purifier and the pressure measured by the third pressure sensor placed in the blood return line exceeds a predetermined value (for example, when coagulation occurs in the blood purifier) in the sixth step of performing forward rotation of the blood pump by a predetermined volume W while opening the first transfusion line and closing the blood removal line, the bypass line for bypassing the blood purifier can be opened.

### Advantageous Effects of Invention

The present invention makes it possible to perform, in a blood purification device in which a transfusion line for supplying a transfusion such as anticoagulant is connected to a blood removal line, automatic blood return without performing reverse rotation of the blood pump and without providing a transfusion supply unit for blood return on a side downstream of the blood pump.

### Brief Description of Drawings

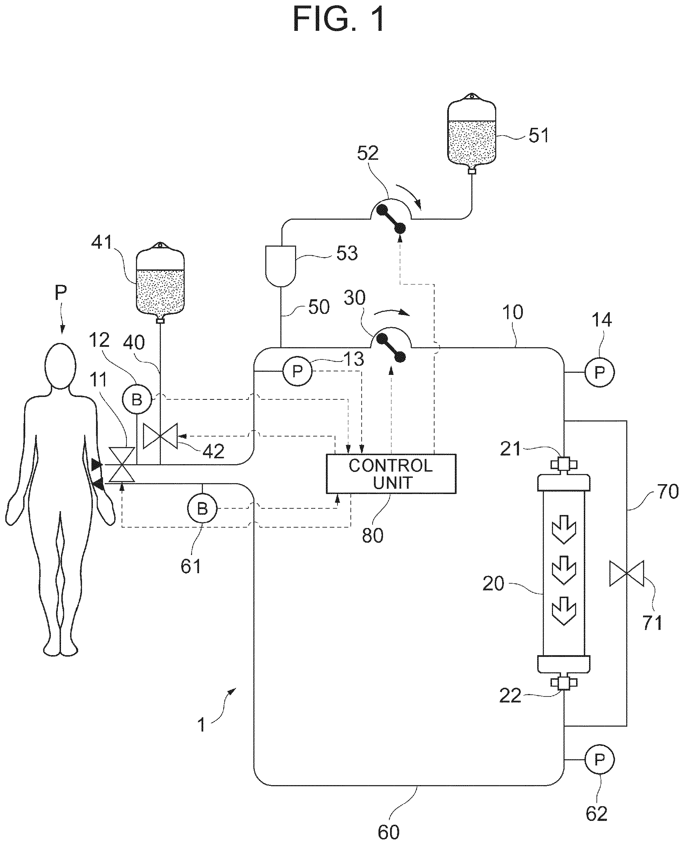

Fig. 1 is a block diagram for describing the entire constitution of a blood purification device according to an embodiment of the present invention.

### Description of Embodiments

Embodiments of the present invention will hereinafter be described referring to the drawings.

First, the constitution of a blood purification device 1 according to the embodiment of the present invention is described referring to Fig. 1.

The blood purification device 1 according to the present embodiment is used for so-called leukocyte apheresis (LCAP) and as shown in Fig. 1, it is equipped with a blood removal line 10, a blood purifier 20, a blood pump 30, a first transfusion line 40, a second transfusion line 50, a blood return line 60, a bypass line 70, and a control unit 80.

The blood removal line 10 is a flow path for delivering the blood taken out from a patient P to the blood purifier 20, while the blood return line 60 is a flow path for returning the blood purified by the blood purifier 20 to the patient P. The blood removal line 10 and the blood return line 60 constitute a blood circuit for circulating the blood taken out from the body of the patient P and returning the blood into the body. The blood removal line 10 has, at the end thereof, a puncture needle not shown in the drawing and via this puncture needle, the blood removal line 10 is connected to the artery of the patient P. The blood return line 60 is connected to the vein of the patient P also via a puncture needle not shown in the drawing.

A second opening/closing valve 11 for opening or closing the blood removal line 10 is placed in the blood removal line 10 on a side upstream of the first transfusion line 40 and the second transfusion line 50. The second opening/closing valve 11 works under the control of the control unit 80 and functions to open or close the blood removal line 10. In the blood removal line 10 on a side upstream of the first transfusion line 40 and the second transfusion line 50, a first air bubble detector 12 for detecting air bubbles contained in the transfusion or blood delivered in the blood removal line 10 is provided. The information relating to the detection results by the first air bubble detector 12 is sent to the control unit 80 and used for the control of the second opening/closing valve 11. In the blood removal line 10 between the blood pump 30 and the patient P, a first pressure sensor 13 for measuring the pressure in the blood removal line 10 is placed, while in the blood removal line 10 between the blood pump 30 and the blood purifier 20, a second pressure sensor 14 is placed. The pressures measured by the first pressure sensor 13 and the second pressure sensor 14 are sent to the control unit 80 and are used for the control of the blood pump 30.

The blood purifier 20 filters out an activated leukocyte contained in the blood introduced from a blood inlet 21 to purify the blood and discharges the purified blood from a blood outlet 22. As the filter, that having an adsorbing material for removing the leukocyte can be used.

The blood pump 30 is placed on the blood removal line 10 as shown in Fig. 1 and functions to deliver the blood of the patient P to the blood purifier 20 via the blood removal line 10 by performing operation (forward rotation) under the control of the control unit 80. When the control unit 80 stops the operation of the blood pump 30, on the other hand, the delivery of the blood in the blood removal line 10 is stopped accordingly.

The first transfusion line 40 is a flow path for supplying a transfusion to the blood removal line 10 on a side upstream of the blood pump 30. To the end of the first transfusion line 40, a transfusion vessel 41 which is a vessel for accumulating the transfusion therein is connected. The first transfusion line 40 is provided with a first opening/closing valve 42 which is driven under the control of the control unit 80 and opens or closes the first transfusion line 40. The transfusion vessel 41 in the present embodiment is placed vertically above the blood removal line 10 so that when the first transfusion line 40 is opened by means of the first opening/closing valve 42, the transfusion moves by gravity flow from the transfusion vessel 41 to the side of the blood removal line 10. In the present embodiment, physiological saline is used as a transfusion to be accumulated in the transfusion vessel 41.

The second transfusion line 50 is a flow path for supplying a second transfusion to the blood removal line 10 on a side upstream of the blood pump 30. To the end of the second transfusion line 50, a second transfusion vessel 51 for accumulating a second transfusion therein is connected. In the present embodiment, the second transfusion line 50 is connected to the blood removal line 10 at a position on a side downstream of the first transfusion line 40. In the present embodiment, an anticoagulant is used as the second transfusion to be accumulated in the second transfusion vessel 51.

The second transfusion line 50 is provided with a transfusion pump 52. The transfusion pump 52 functions to deliver a second transfusion to the side of the blood removal line 10 via the second transfusion line 50 by operating (performing forward operation of) the transfusion pump under the control of the control unit 80. When the control unit 80 stops the operation of the transfusion pump 52, on the other hand, the delivery of the second transfusion in the second transfusion line 50 is stopped. The second transfusion line 50 has, on the side downstream of the transfusion pump 52, a chamber 53. The chamber 53 functions to prevent air bubbles flowing in the second transfusion line 50 from entering the blood removal line 10.

The blood return line 60 is provided with a second air bubble detector 61 for detecting air bubbles contained in the transfusion or blood delivered in the blood return line 60. The information relating to the detection results by the second air bubble detector 61 is sent to the control unit 80 and is used for the control of the blood pump 30. In the blood return line 60, a third pressure sensor 62 for measuring the pressure in the blood return line 60 is placed. The pressure measured by the third pressure sensor 62 is sent to the control unit 80 and is used for the control of the blood pump 30.

A bypass line 70 is a flow path for delivering the blood from the blood removal line 10 to the blood return line 60 not via the blood purifier 20 (for bypassing the blood purifier 20). The bypass line 70 is provided with a third opening/closing valve 71 which works under the control of the control unit 80 and opens or closes the bypass line 70.

The control unit 80 is comprised of, for example, a computer equipped with a memory, CPU (Central Processing Unit), and various programs loaded in the memory are performed by CPU to control various units of the blood purification device 1.

The control unit 80 in the present embodiment controls the blood pump 30, the transfusion pump 52, the first opening/closing valve 42, the second opening/closing valve 11, and the third opening/closing valve 71 to achieve automatic return of the blood to the patient P. More specifically, the control unit 80 achieves automatic blood return to the upstream side by successively performing the following steps: a first step of stopping the operation of the blood pump 30 and the transfusion pump 52 and at the same time, opening the first transfusion line 40 by means of the first opening/closing valve 42 and closing the blood removal line 10 by means of the second opening/closing valve 11, a second step of performing forward rotation of the blood pump 30 by a predetermined volume X to move the first transfusion from the first transfusion line 40 to the blood removal line 10, a third step of performing reverse rotation of the transfusion pump 52 by a predetermined volume Y to move the first transfusion in the blood removal line 10 to the second transfusion line 50, a fourth step of closing the first transfusion line 40 by means of the first opening/closing valve 42 and opening the blood removal line 10 by means of the second opening/closing valve 11, and a fifth step of performing forward rotation of the transfusion pump 52 by a predetermined volume Z to move the first transfusion in the second transfusion line 50 to the blood removal line 10 and return the first transfusion to the patient P. In the above description, the predetermined volume X can be set at an internal volume or more of a portion of the blood removal line 10 positioned between the first transfusion line 40 and the second transfusion line 50. The predetermined volume Y can be set at an internal volume or more of a portion of the blood removal line 10 positioned between the patient-side end of the blood removal line 10 and the first transfusion line 40. The predetermined volume Z can be set at an internal volume or more of a portion of the blood removal line 10 positioned between the patient-side end of the blood removal line 10 and the first transfusion line 40.

The control unit 80 achieves automatic blood return to the downstream side by performing, before the first step or after the fifth step, a sixth step of performing forward rotation of the blood pump 30 by a predetermined volume W while opening the first transfusion line 40 by means of the first opening/closing valve 42 and closing the blood removal line 10 by means of the second opening/closing valve 11. The predetermined volume W can be set at the sum or more of the internal volume of a portion of the blood removal line 10 on a side downstream of the second transfusion line 50, the internal volume of the blood purifier 20, and the internal volume of the blood return line 60.

In performing the fifth step, the control unit 80 stops the transfusion pump 52 (and/or closes the blood removal line 10 by means of the second opening/closing valve 11) when the first air bubble detector 12 detects air bubbles. The control unit 80 can perform the sixth step when the first air bubble detector 12 detects air bubbles in the fifth step or the pressure measured by the first pressure sensor 13 exceeds a predetermined value in the fifth step.

When the second air bubble detector 61 detects air bubbles in the sixth step or the pressure measured by the third pressure sensor 62 exceeds a predetermined value in the sixth step, the control unit 80 can terminate the operation (stop the blood pump 30 and the like). When the pressure measured by the second pressure sensor 14 exceeds a predetermined value in the sixth step or a difference between the pressure measured by the second pressure sensor 14 and the pressure measured by the third pressure sensor 62 exceeds a predetermined value in the sixth step, the control unit 80 can open the bypass line 70 by means of the third opening/closing valve 71.

### <Automatic blood return to upstream side>

First, automatic blood return to the upstream side will be described. The control unit 80 stops the operation of the blood pump 30 and the transfusion pump 52 and at the same time,opens the first transfusion line 40 by means of the first opening/closing valve 42 and closes the blood removal line 10 by means of the second opening/closing valve 11 (first step). Then the control unit 80 moves the first transfusion from the first transfusion line 40 to the blood removal line 10 by performing forward rotation of the blood pump 30 by the predetermined volume X (second step). The control unit 80 then moves the first transfusion in the blood removal line 10 to the second transfusion line 50 by stopping the blood pump 30 and performing reverse rotation of the transfusion pump 52 by the predetermined volume Y (third step).

Then the control unit 80 closes the first transfusion line 40 by means of the first opening/closing valve 42 and opens the blood removal line 10 by means of the second opening/closing valve 11 (fourth step). The control unit 80 then performs forward rotation of the transfusion pump 52 by the predetermined volume Z to move the first transfusion in the second transfusion line 50 to the blood removal line 10 and return the first transfusion to the patient P (fifth step). The control unit 80 can achieve automatic blood return to the upstream side by successively performing these first to fifth steps. In performing the fifth step, the control unit 80 can stop the transfusion pump 52 (and/or close the blood removal line 10 by means of the second opening/closing valve 11) when the first air bubble detector 12 detects air bubbles.

### <Automatic blood return to downstream side>

Next, automatic blood return to the downstream side will be described. When the first air bubble detector 12 detects air bubbles in the fifth step or the pressure measured by the first pressure sensor 13 exceeds a predetermined value in the fifth step, the control unit 80 opens the first transfusion line 40 by means of the first opening/closing valve 42 and at the same time, performs forward rotation of the blood pump 30 by the predetermined volume W while closing the blood removal line 10 by means of the second opening/closing valve 11 (sixth step). The control unit 80 can achieve automatic blood return to the downstream side by this sixth step. This sixth step may be performed before the first step.

The control unit 80 can terminate the operation (in other words, stop the blood pump 30) when the second air bubble detector 61 detects air bubbles in the sixth step or the pressure measured by the third pressure sensor 62 exceeds a predetermined value in the sixth step. The control unit 80 can open the bypass line 70 by means of the third opening/closing valve 71 when the pressure measured by the second pressure sensor 14 exceeds a predetermined value in the sixth step or a difference between the pressure measured by the second pressure sensor 14 and the pressure measured by the third pressure sensor 62 exceeds a predetermined value in the sixth step.

In the blood purification device 1 according to the embodiment described above, the control unit 80 can achieve automatic blood return to a patient by controlling the blood pump 30, the transfusion pump 52, the first opening/closing valve 42, and the second opening/closing valve 11 in a specific manner. Described specifically, the control unit can achieve automatic blood return to the patient P by successively performing the following steps: (1) stopping the operation of the blood pump 30 and the transfusion pump 52 and at the same time, opening the first transfusion line 40 and closing the blood removal line 10, (2) performing forward rotation of the blood pump 30 by the predetermined volume X to move the first transfusion from the first transfusion line 40 to the blood removal line 10, (3) performing reverse rotation of the transfusion pump 52 by the predetermined volume Y to move the first transfusion in the blood removal line 10 to the second transfusion line 50, (4) closing the first transfusion line 40 and opening the blood removal line 10 , and (5) performing forward rotation of the transfusion pump 52 by the predetermined volume Z to move the first transfusion in the second transfusion line 50 to the blood removal line 10 and return the first transfusion to the patient P. At this time, instead of the second transfusion, the first transfusion moved temporarily to the second transfusion line 50 can be returned to the patient P so that the second transfusion can be prevented from entering the body of the patient P. In addition, since the forward rotation volume (X) of the blood pump 30, the reverse rotation volume (Y) of the transfusion pump 52, and the forward rotation volume (Z) of the transfusion pump 52 are specified respectively, the amount of the blood to be returned to the patient P can be controlled precisely.

In the blood purification device 1 according to the embodiment described above, when in moving the first transfusion in the second transfusion line 50 to the blood removal line 10 and returning the first transfusion to the patient P by performing forward rotation of the transfusion pump 52 by the predetermined volume Z, the first air bubble detector 12 provided on the side upstream of the first transfusion line 40 and the second transfusion line 50 detects air bubbles, the transfusion pump 52 can be terminated (and/or the blood removal line 10 can be closed). This makes it possible to prevent the transfusion or blood to be returned to the patient P from containing air bubbles.

In the blood purification device 1 according to the embodiment described above, before the first step of stopping the operation of the blood pump 30 and the transfusion pump 52 or after the fifth step of performing forward rotation of the transfusion pump 52 by the predetermined volume Z to move the first transfusion in the second transfusion line 50 to the blood removal line 10 and return the first transfusion to the patient P, the sixth step of performing forward rotation of the blood pump 30 by the predetermined volume W while opening the first transfusion line 40 and closing the blood removal line 10 can be performed. In short, before blood return via the blood removal line 10 or after sufficient return of the transfusion to the patient P via the blood removal line 10, blood return via the blood return line 60 can be performed.

In the blood purification device 1 according to the embodiment described above, when the first air bubble detector 12 provided in the blood removal line 10 detects air bubbles in the fifth step of performing forward rotation of the transfusion pump 52 by the predetermined volume Z to move the first transfusion in the second transfusion line 50 to the blood removal line 10 and return the first transfusion to the patient P, it is possible to proceed to the sixth step of performing forward rotation of the blood pump 30 by the predetermined volume W while opening the first transfusion line 40 and closing the blood removal line 10. This makes it possible to prevent the transfusion to be returned to the patient P from containing air bubbles and at the same time, to proceed to the sixth step without requiring an additional operation by an operator.

In the blood purification device 1 according to the embodiment described above, when the second air bubble detector 61 provided in the blood return line 60 detects air bubbles in the sixth step of performing forward rotation of the blood pump 30 by the predetermined volume W while opening the first transfusion line 40 and closing the blood removal line 10, the operation can be terminated. This therefore makes it possible to prevent the blood to be returned to the patient P via the blood return line 60 from containing air bubbles.

In the blood purification device 1 according to the embodiment described above, when the pressure measured by the first pressure sensor 13 placed between the blood pump 30 on the blood removal line 10 and the patient P exceeds a predetermined value in the fifth step of performing forward rotation of the transfusion pump 52 by the predetermined volume Z to move the first transfusion in the second transfusion line 50 to the blood removal line 10 and return the first transfusion to the patient P, it is possible to proceed to the sixth step of performing forward rotation of the blood pump 30 by the predetermined volume W while opening the first transfusion line 40 and closing the blood removal line 10. In short, it is possible to switch to blood return via the blood return line 60 without forcibly pouring the transfusion when the connection part with the patient P is clogged with a clot.

In the blood purification device 1 according to the embodiment described above, when the pressure measured by the second pressure sensor 14 placed between the blood pump 30 on the blood removal line 10 and the blood purifier 20 exceeds a predetermined value (for example, when coagulation occurs in the blood purifier 20) in the sixth step of performing forward rotation of the blood pump 30 by the predetermined volume W while opening the first transfusion line 40 and closing the blood removal line 10, the bypass line 70 for bypassing the blood purifier 20 can be opened.

In the blood purification device 1 according to the embodiment described above, when the pressure measured by the third pressure sensor 62 placed in the blood return line 60 exceeds a predetermined value (for example, when a connection part with the patient P is clogged with a clot and further blood return is prevented) in the sixth step of performing forward rotation of the blood pump 30 by the predetermined volume W while opening the first transfusion line 40 and closing the blood removal line 10, the operation can be terminated.

In the blood purification device 1 according to the embodiment described above, when a difference between the pressure measured by the second pressure sensor 14 placed between the blood pump 30 on the blood removal line 10 and the blood purifier 20 and the pressure measured by the third pressure sensor 62 placed in the blood return line 60 exceeds a predetermined value in the sixth step of performing forward rotation of the blood pump 30 by the predetermined volume W while opening the first transfusion line 40 and closing the blood removal line 10 (for example, when coagulation occurs in the blood purifier 20), the bypass line 70 for bypassing the blood purifier 20 can be opened.

In the present embodiment, an example of placing the second transfusion line 50 on the side downstream of the first transfusion line 40 is shown, but the second transfusion line 50 may be placed on the side upstream of the first transfusion line 40.

In the present embodiment, shown is an application example of the present invention to a blood purification device used for leukocyte apheresis (LCAP). The present invention can also be applied to another blood purification device having a similar constitution (blood removal line, blood pump, first transfusion line, transfusion vessel, first opening/closing valve, second transfusion line, second opening/closing valve, transfusion pump ). For example, the present invention can also be applied to a blood purification device equipped with a blood purifier for filtration or diffusion to be used for plasma exchange (PE), double filtration plasmapheresis (DFPP), continuous hemofiltration (CHF), continuous hemodialysis (CHD), or continuous hemodiafiltration (CHDF).

### Reference Signs List

- 1:: Blood purification device

- 10:: Blood removal line
- 11:: Second opening/closing valve
- 12:: First air bubble detector
- 13:: First pressure sensor
- 14:: Second pressure sensor
- 20:: Blood purifier
- 30:: Blood pump
- 40:: First transfusion line
- 41:: Transfusion vessel
- 42:: First opening/closing valve
- 50:: Second transfusion line
- 52:: Transfusion pump
- 53:: Chamber
- 60: Blood return line
- 61:: Second air bubble detector
- 62:: Third pressure sensor
- 70:: Bypass line
- 71:: Third opening/closing valve
- 80:: Control unit
- P:: Patient

## Claims

1. A blood purification device (1) comprising: a blood removal line (10) for delivering a blood taken out from a patient to a blood purifier (20); a blood pump (30) placed on the blood removal line (10), **characterized in that** the blood purification device (1) further comprises a first transfusion line (40) for supplying a first transfusion to the blood removal line (10) on a side upstream of the blood pump (30); a transfusion vessel (41) connected to the first transfusion line (40); a first opening/closing valve for opening or closing the first transfusion line (40); a second transfusion line (50) for supplying a second transfusion to the blood removal line (10) on a side upstream of the blood pump (30); a transfusion pump (52) placed on the second transfusion line (50); a second opening/closing valve (11) placed in the blood removal line (10) on a side upstream of the first transfusion line (40) and the second transfusion line (50) for opening or closing the blood removal line (10); and a control unit (80) for controlling the blood pump (30), the transfusion pump (52), the first opening/closing valve (42) and the second opening/closing valve (11),
wherein the control unit (80) achieves automatic return of the blood to the patient by successively performing the following steps of: a first step of stopping operation of the blood pump (30) and the transfusion pump (52), and opening the first transfusion line (40) by means of the first opening/closing valve (42) and closing the blood removal line (10) by means of the second opening/closing valve (11); a second step of performing forward rotation of the blood pump (30) by a predetermined volume X to move the first transfusion from the first transfusion line (40) to the blood removal line (10); a third step of performing reverse rotation of the transfusion pump (52) by a predetermined volume Y to move the first transfusion in the blood removal line (10) to the second transfusion line (50); a fourth step of closing the first transfusion line by means of the first opening/closing valve (42) and opening the blood removal line (10) by the second opening/closing valve (11); and a fifth step of performing forward rotation of the transfusion pump (52) by a predetermined volume Z to move the first transfusion in the second transfusion (50) line to the blood removal line (10) and return the first transfusion to the patient.

2. The blood purification device (1) according to Claim 1, wherein the first transfusion line (40) is connected to the blood removal line (10) on a side upstream of the second transfusion line (50).

3. The blood purification device (1) according to Claim 1, wherein the first transfusion line (40) is connected to the blood removal line (10) on a side downstream of the second transfusion line (50).

4. The blood purification device (1) according to any one of Claims 1 to 3, further comprising a chamber in the second transfusion line (50) on a side downstream of the transfusion pump (52).

5. The blood purification device (1) according to any one of Claims 1 to 4, wherein the predetermined volume X is an internal volume or more of a portion of the blood removal line (10) positioned between the first transfusion line (10) and the second transfusion line (50).

6. The blood purification device (1) according to any one of Claims 1 to 5, wherein the predetermined volume Y is an internal volume or more of a portion of the blood removal line (10) positioned between a patient-side end thereof and either one of the first transfusion line (40) or the second transfusion line (50) present on an upstream side.

7. The blood purification device (1) according to any one of Claims 1 to 6, wherein the predetermined volume Z is an internal volume or more of a portion of the blood removal line (10) positioned between a patient-side end thereof and either one of the first transfusion line (40) or the second transfusion line (50) present on an upstream side.

8. The blood purification device (1) according to any one of Claims 1 to 7, further comprising a first air bubble detector (12) provided in the blood removal line (10) on a side upstream of the first transfusion line (40) and the second transfusion line (50),
wherein in performing the fifth step, the control unit (80) stops the transfusion pump (52) and/or closes the blood removal line (10) by means of the second opening/closing valve (11) when the first air bubble detector (12) detects an air bubble.

9. The blood purification device (1) according to any one of Claims 1 to 8, further comprising a blood return line (60) for returning the blood purified by the blood purifier (20) to the patient,
wherein the control unit (80) performs, before the first step or after the fifth step, a sixth step of performing forward rotation of the blood pump (30) by a predetermined volume W while opening the first transfusion line (40) by means of the first opening/closing valve (42) and closing the blood removal line (10) by means of the second opening/closing valve (11).

10. The blood purification device (1) according to Claim 9, wherein the predetermined amount W is a sum or more of an internal volume of a portion of the blood removal line (10) on a side downstream of the second transfusion line (50), an internal volume of the blood purifier (20), and an internal volume of the blood return line (60).

11. The blood purification device (1) according to Claim 9 or 10, further comprising a first air bubble detector (12) provided in the blood removal line (10) on a side upstream (40) of the first transfusion line and the second transfusion line (50),
wherein the control unit (80) performs the sixth step when the first air bubble detector (12) detects an air bubble in the fifth step.

12. The blood purification device (1) according to any one of Claims 9 to 11, further comprising a second air bubble detector (61) provided in the blood return line (60),
wherein the control unit (80) terminates operation when the second air bubble detector (61) detects an air bubble in the sixth step.

13. The blood purification device (1) according to any one of Claims 9 to 12, further comprising a first pressure sensor (13) placed between the blood pump (30) on the blood removal line (10) and the patient for measuring a pressure in the blood removal line (10),
wherein the control unit (80) performs the sixth step when a pressure measured by the first pressure sensor (13) exceeds a predetermined value in the fifth step.

14. The blood purification device (1) according to Claim 13, further comprising:
a bypass line (70) for bypassing the blood purifier (20),
a third opening/closing valve (71) for opening or closing the bypass line (70), and
a second pressure sensor (14) placed between the blood pump (30) and the blood purifier (20),
wherein the control unit (80) opens the bypass line (70) by means of the third opening/closing valve (71) when a pressure measured by the second pressure sensor (14) exceeds a predetermined value in the sixth step.

15. The blood purification device (1) according to Claim 14, further comprising a third pressure sensor (62) for measuring a pressure in the blood return line (60),
wherein the control unit terminates operation when a pressure measured by the third pressure sensor (62) exceeds a predetermined value in the sixth step.

16. The blood purification device (1) according to Claim 15, further comprising:
a bypass line (70) for bypassing the blood purifier (20), and
a third opening/closing valve (71) for opening or closing the bypass line (70),
wherein the control unit (80) opens the bypass line (70) by means of the third opening/closing valve (71) when a difference between the pressure measured by the second pressure sensor (14) and the pressure measured by the third pressure sensor (62) exceeds a predetermined value in the sixth step.

17. The blood purification device (1) according to any one of Claims 1 to 16, wherein:
the first transfusion is physiological saline, and
the second transfusion is different from physiological saline.

18. The blood purification device (1) according to Claim 17, wherein the second transfusion contains an anticoagulant.

## Patentansprüche

1. Blutreinigungsvorrichtung (1), umfassend: eine Blutentnahmeleitung (10) zum Abgeben von Blut, das einem Patienten entnommen wurde, an einen Blutreiniger (20); eine Blutpumpe (30), die in die Blutentnahmeleitung (10) geschaltet ist, **dadurch gekennzeichnet, dass** die Blutreinigungsvorrichtung (1) weiterhin umfasst: eine erste Transfusionsleitung (40) zum Zuführen einer ersten Transfusion zu der Blutentnahmeleitung (10) auf einer Seite vor der Blutpumpe (30); ein Transfusionsgefäß (41), das an die erste Transfusionsleitung (40) angeschlossen ist; ein erstes Öffnungs- und Schließventil zum Öffnen oder Schließen der ersten Transfusionsleitung (40); eine zweite Transfusionsleitung (50) zum Zuführen einer zweiten Transfusion zu der Blutentnahmeleitung (10) auf einer Seite vor der Blutpumpe (30); eine Transfusionspumpe (52), die in die zweite Transfusionsleitung (50) geschaltet ist; ein zweites Öffnungs- und Schließventil (11), das auf einer Seite vor der ersten Transfusionsleitung (40) und der zweiten Transfusionsleitung (50) in die Blutentnahmeleitung (10) geschaltet ist, zum Öffnen oder Schließen der Blutentnahmeleitung (10); und eine Steuereinheit (80) zum Steuern der Blutpumpe (30), der Transfusionspumpe (52), des ersten Öffnungs- und Schließventils (42) und des zweiten Öffnungs- und Schließventils (11),
wobei die Steuereinheit (80) eine automatische Rückführung des Bluts des Patienten erreicht, indem sie nacheinander die folgenden Schritte durchführt: einen ersten Schritt, in dem der Betrieb der Blutpumpe (30) und der Transfusionspumpe (52) abgebrochen wird und die erste Transfusionsleitung (40) mittels des ersten Öffnungs- und Schließventils (42) geöffnet wird und die Blutentnahmeleitung (10) mittels des zweiten Öffnungs- und Schließventils (11) geschlossen wird; einen zweiten Schritt, in dem die Vorwärtsrotation der Blutpumpe (30) um ein vorbestimmtes Volumen X durchgeführt wird, um die erste Transfusion aus der ersten Transfusionsleitung (40) in die Blutentnahmeleitung (10) zu bewegen; einen dritten Schritt, in dem die Rückwärtsrotation der Transfusionspumpe (52) um ein vorbestimmtes Volumen Y durchgeführt wird, um die erste Transfusion in der Blutentnahmeleitung (10) in die zweite Transfusionsleitung (50) zu bewegen; einen vierten Schritt, in dem die erste Transfusionsleitung mittels des ersten Öffnungs- und Schließventils (42) geschlossen wird und die Blutentnahmeleitung (10) mittels des zweiten Öffnungs- und Schließventils (11) geöffnet wird; und einen fünften Schritt, in dem die Vorwärtsrotation der Transfusionspumpe (52) um ein vorbestimmtes Volumen Z durchgeführt wird, um die erste Transfusion in der zweiten Transfusionsleitung (50) in die Blutentnahmeleitung (10) zu bewegen und die erste Transfusion zum Patienten zurückzuführen.

2. Blutreinigungsvorrichtung (1) gemäß Anspruch 1, wobei die erste Transfusionsleitung (40) auf einer Seite vor der zweiten Transfusionsleitung (50) an die Blutentnahmeleitung (10) angeschlossen ist.

3. Blutreinigungsvorrichtung (1) gemäß Anspruch 1, wobei die erste Transfusionsleitung (40) auf einer Seite hinter der zweiten Transfusionsleitung (50) an die Blutentnahmeleitung (10) angeschlossen ist.

4. Blutreinigungsvorrichtung (1) gemäß einem der Ansprüche 1 bis 3, weiterhin umfassend eine Kammer in der zweiten Transfusionsleitung (50) auf einer Seite hinter der Transfusionspumpe (52).

5. Blutreinigungsvorrichtung (1) gemäß einem der Ansprüche 1 bis 4, wobei das vorbestimmte Volumen X dem Innenvolumen eines Teils der Blutentnahmeleitung (10), der sich zwischen der ersten Transfusionsleitung (10) und der zweiten Transfusionsleitung (50) befindet, oder mehr entspricht.

6. Blutreinigungsvorrichtung (1) gemäß einem der Ansprüche 1 bis 5, wobei das vorbestimmte Volumen Y dem Innenvolumen eines Teils der Blutentnahmeleitung (10), der sich zwischen dem Ende auf deren Patientenseite und entweder der ersten Transfusionsleitung (40) oder der zweiten Transfusionsleitung (50) befindet und vorgeschaltet ist, oder mehr entspricht.

7. Blutreinigungsvorrichtung (1) gemäß einem der Ansprüche 1 bis 6, wobei das vorbestimmte Volumen Z dem Innenvolumen eines Teils der Blutentnahmeleitung (10), der sich zwischen dem Ende auf deren Patientenseite und entweder der ersten Transfusionsleitung (40) oder der zweiten Transfusionsleitung (50) befindet und nachgeschaltet ist, oder mehr entspricht.

8. Blutreinigungsvorrichtung (1) gemäß einem der Ansprüche 1 bis 7, weiterhin umfassend einen ersten Luftblasendetektor (12), der sich in der ersten Blutentnahmeleitung (10) auf einer Seite, die der ersten Transfusionsleitung (40) und der zweiten Transfusionsleitung (50) vorgeschaltet ist, befindet,
wobei bei der Durchführung des fünften Schritts die Steuereinheit (80) die Transfusionspumpe (52) abschaltet und/oder die Blutentnahmeleitung (10) mittels des zweiten Öffnungs- und Schließventils (11) schließt, wenn der erste Luftblasendetektor (12) eine Luftblase nachweist.

9. Blutreinigungsvorrichtung (1) gemäß einem der Ansprüche 1 bis 8, weiterhin umfassend eine Blutrückführungsleitung (60) zum Rückführen des durch den Blutreiniger (20) gereinigten Bluts zum Patienten,
wobei die Steuereinheit (80) vor dem ersten Schritt oder nach dem fünften Schritt einen sechsten Schritt durchführt, in dem eine Vorwärtsrotation der Blutpumpe (30) um ein vorbestimmtes Volumen W durchgeführt wird, während die erste Transfusionsleitung (40) mittels des ersten Öffnungsund Schließventils (42) geöffnet wird und die Blutentnahmeleitung (10) mittels des zweiten Öffnungs- und Schließventils (11) geschlossen wird.

10. Blutreinigungsvorrichtung (1) gemäß Anspruch 9, wobei die vorbestimmte Menge W der Summe des Innenvolumens eines Teils der Blutentnahmeleitung (10) auf einer Seite hinter der zweiten Transfusionsleitung (50), des Innenvolumens des Blutreinigers (20) und des Innenvolumens der Blutrückführungsleitung (60) oder mehr entspricht.

11. Blutreinigungsvorrichtung (1) gemäß Anspruch 9 oder 10, weiterhin umfassend einen ersten Luftblasendetektor (12), der sich in der Blutentnahmeleitung (10) auf einer Seite, die der ersten Transfusionsleitung (40) und der zweiten Transfusionsleitung (50) vorgeschaltet ist, befindet,
wobei die Steuereinheit (80) den sechsten Schritt durchführt, wenn der erste Luftblasendetektor (12) im fünften Schritt eine Luftblase nachweist.

12. Blutreinigungsvorrichtung (1) gemäß einem der Ansprüche 9 bis 11, weiterhin umfassend einen zweiten Luftblasendetektor (61), der sich in der Blutrückführungsleitung (60) befindet,
wobei die Steuereinheit (80) den Betrieb beendet, wenn der zweite Luftblasendetektor (61) im sechsten Schritt eine Luftblase nachweist.

13. Blutreinigungsvorrichtung (1) gemäß einem der Ansprüche 9 bis 12, weiterhin umfassend einen ersten Drucksensor (13), der zwischen der Blutpumpe (30) in der Blutentnahmeleitung (10) und dem Patienten platziert ist, um einen Druck in der Blutentnahmeleitung (10) zu messen,
wobei die Steuereinheit (80) den sechsten Schritt durchführt, wenn ein durch den ersten Drucksensor (13) gemessener Druck im fünften Schritt einen vorbestimmten Wert überschreitet.

14. Blutreinigungsvorrichtung (1) gemäß Anspruch 13, weiterhin umfassend:
eine Umleitung (70) zum Umgehen des Blutreinigers (20),
ein drittes Öffnungs- und Schließventil (71) zum Öffnen oder Schließen der Umleitung (70) und
einen zweiten Drucksensor (14), der zwischen der Blutpumpe (30) und dem Blutreiniger (20) platziert ist,
wobei die Steuereinheit (80) mittels des dritten Öffnungs- und Schließventils (71) die Umleitung (70) öffnet, wenn ein durch den zweiten Drucksensor (14) gemessener Druck im sechsten Schritt einen vorbestimmten Wert überschreitet.

15. Blutreinigungsvorrichtung (1) gemäß Anspruch 14, weiterhin umfassend einen dritten Drucksensor (62) zum Messen eines Drucks in der Blutrückführungsleitung (60),
wobei die Steuereinheit den Betrieb beendet, wenn ein durch den dritten Drucksensor (62) gemessener Druck im sechsten Schritt einen vorbestimmten Wert überschreitet.

16. Blutreinigungsvorrichtung (1) gemäß Anspruch 15, weiterhin umfassend:
eine Umleitung (70) zum Umgehen des Blutreinigers (20) und
ein drittes Öffnungs- und Schließventil (71) zum Öffnen oder Schließen der Umleitung (70),
wobei die Steuereinheit (80) mittels des dritten Öffnungs- und Schließventils (71) die Umleitung (70) öffnet, wenn die Differenz zwischen dem durch den zweiten Drucksensor (14) gemessenen Druck und dem durch den dritten Drucksensor (62) gemessenen Druck im sechsten Schritt einen vorbestimmten Wert überschreitet.

17. Blutreinigungsvorrichtung (1) gemäß einem der Ansprüche 1 bis 16, wobei:
es sich bei der ersten Transfusion um physiologische Kochsalzlösung handelt und
die zweite Transfusion keine physiologische Kochsalzlösung ist.

18. Blutreinigungsvorrichtung (1) gemäß Anspruch 17, wobei die zweite Transfusion ein Antikoagulationsmittel enthält.

## Revendications

1. Dispositif de purification de sang (1) comprenant : une ligne de retrait de sang (10) pour délivrer un sang prélevé auprès d'un patient à un purificateur de sang (20) ; une pompe à sang (30) placée sur la ligne de retrait de sang (10), **caractérisé en ce que** le dispositif de purification de sang (1) comprend en outre une première ligne de transfusion (40) pour délivrer une première transfusion vers la ligne de retrait de sang (10) sur un côté amont de la pompe à sang (30) ; un récipient de transfusion (41) relié à la première ligne de transfusion (40) ; une première vanne de marche/arrêt pour ouvrir ou fermer la première ligne de transfusion (40) ; une seconde ligne de transfusion (50) pour fournir une seconde transfusion vers la ligne de retrait de sang (10) sur un côté amont de la pompe à sang (30) ; une pompe à transfusion (52) placée sur la seconde ligne de transfusion (50) ; une deuxième vanne de marche/arrêt (11) placée dans la ligne de retrait de sang (10) sur un côté amont de la première ligne de transfusion (40) et de la seconde ligne de transfusion (50) pour ouvrir ou fermer la ligne de retrait de sang (10) ; et une unité de commande (80) pour commander la pompe à sang (30), la pompe à transfusion (52), la première vanne de marche/arrêt (42) et la deuxième vanne de marche/arrêt (11),
dans lequel l'unité de commande (80) réalise un retour automatique du sang au patient en effectuant successivement les étapes suivantes : une première étape consistant à arrêter le fonctionnement de la pompe à sang (30) et de la pompe à transfusion (52), et ouvrir la première ligne de transfusion (40) au moyen de la première vanne de marche/arrêt (42) et fermer la ligne de retrait de sang (10) au moyen de la deuxième vanne de marche/arrêt (11) ; une deuxième étape consistant en une rotation vers l'avant de la pompe à sang (30) d'une quantité prédéterminée X pour déplacer la première transfusion depuis la première ligne de transfusion (40) vers la ligne de retrait de sang (10) ; une troisième étape consistant en une rotation en sens inverse de la pompe à transfusion (52) d'une quantité prédéterminée Y pour déplacer la première transfusion dans la ligne de retrait de sang (10) vers la seconde ligne de transfusion (50) ; une quatrième étape consistant à fermer la première ligne de transfusion par l'intermédiaire de la première vanne de marche/arrêt (42) et ouvrir la ligne de retrait de sang (10) par l'intermédiaire de la deuxième vanne de marche/arrêt (11); et une cinquième étape consistant en une rotation de la pompe à transfusion (52) vers l'avant d'une quantité prédéterminée Z afin de déplacer la première transfusion dans la seconde ligne de transfusion (50) vers la ligne de retrait de sang (10) et à retourner la première transfusion vers le patient.

2. Dispositif de purification de sang (1) selon la revendication 1, dans lequel la première ligne de transfusion (40) est reliée à la ligne de retrait de sang (10) sur un côté amont de la seconde ligne de transfusion (50).

3. Dispositif de purification de sang (1) selon la revendication 1, dans lequel la première ligne de transfusion (40) est reliée à la ligne de retrait de sang (10) sur un côté aval de la seconde ligne de transfusion (50).

4. Dispositif de purification de sang (1) selon l'une quelconque des revendications 1 à 3, comprenant en outre une chambre dans la seconde ligne de transfusion (50) sur un côté aval de la pompe à transfusion (52).

5. Dispositif de purification de sang (1) selon l'une quelconque des revendications 1 à 4, dans lequel la quantité prédéterminée X est un volume interne ou plus d'une partie de la ligne de retrait de sang (10) positionnée entre la première ligne de transfusion (40) et la seconde ligne de transfusion (50).

6. Dispositif de purification de sang (1) selon l'une quelconque des revendications 1 à 5, dans lequel la quantité prédéterminée Y est un volume interne ou plus d'une partie de la ligne de retrait de sang (10) positionnée entre une extrémité côté patient de celle-ci et l'une quelconque de la première ligne de transfusion (40) ou de la seconde ligne de transfusion (50) présente sur un côté amont.

7. Dispositif de purification de sang (1) selon l'une quelconque des revendications 1 à 6, dans lequel la quantité prédéterminée Z est un volume interne ou plus d'une partie de la ligne de retrait de sang (10) positionnée entre une extrémité côté patient de celle-ci et l'une quelconque de la première ligne de transfusion (40) ou de la seconde ligne de transfusion (50) présente sur un côté aval.

8. Dispositif de purification de sang (1) selon l'une quelconque des revendications 1 à 7, comprenant en outre un premier détecteur de bulle d'air (12) prévu dans la ligne de retrait de sang (10) sur un côté amont de la première ligne de transfusion (40) et de la seconde ligne de transfusion (50),
dans lequel, lors de l'exécution de la cinquième étape, l'unité de commande (80) arrête la pompe à transfusion (52) et/ou ferme la ligne de retrait de sang (10) au moyen de la deuxième vanne de marche/arrêt (11) lorsque le premier détecteur de bulle d'air (12) détecte une bulle d'air.

9. Dispositif de purification de sang (1) selon l'une quelconque des revendications 1 à 8, comprenant en outre une ligne de retour de sang (60) pour un retour du sang purifié par le purificateur de sang (20) au patient,
dans lequel l'unité de commande (80) effectue, avant la première étape ou après la cinquième étape, une sixième étape consistant en une rotation vers l'avant de la pompe à sang (30) d'une quantité prédéterminée W tout en ouvrant la première ligne de transfusion (40) au moyen de la première vanne de marche/arrêt (42) et en fermant la ligne de retrait de sang (10) au moyen de la deuxième vanne de marche/arrêt (11).

10. Dispositif de purification de sang (1) selon la revendication 9, dans lequel la quantité prédéterminée W est une somme ou plus d'un volume interne d'une partie de la ligne de retrait de sang (10) sur un côté aval de la seconde ligne de transfusion (50), d'un volume interne du purificateur de sang (20), et d'un volume interne de la ligne de retour de sang (60).

11. Dispositif de purification de sang (1) selon la revendication 9 ou 10, comprenant en outre un premier détecteur de bulle d'air (12) prévu dans la ligne de retrait de sang (10) sur un côté amont de la première ligne de transfusion (40) et de la seconde ligne de transfusion (50),
dans lequel l'unité de commande (80) effectue la sixième étape lorsque le premier détecteur de bulle d'air (12) détecte une bulle d'air dans la cinquième étape.

12. Dispositif de purification de sang (1) selon l'une quelconque des revendications 9 à 11, comprenant en outre un second détecteur de bulle d'air (61) prévu dans la ligne de retour de sang (60),
dans lequel l'unité de commande (80) met fin au fonctionnement lorsque le second détecteur de bulle d'air (61) détecte une bulle d'air dans la sixième étape.

13. Dispositif de purification sanguine (1) selon l'une quelconque des revendications 9 à 12, comprenant en outre un premier capteur de pression (13) placé entre la pompe à sang (30) sur la ligne de retrait de sang (10) et le patient pour mesurer une pression dans la ligne de retrait de sang (10),
dans lequel l'unité de commande (80) effectue la sixième étape lorsqu'une pression mesurée par le premier capteur de pression (13) dépasse une valeur prédéterminée dans la cinquième étape.

14. Dispositif de purification de sang (1) selon la revendication 13, comprenant en outre :
une ligne de dérivation (70) pour contourner le purificateur de sang (20),
une troisième vanne de marche/arrêt (71) pour ouvrir ou fermer la ligne de dérivation (70), et
un deuxième capteur de pression (14) placé entre la pompe à sang (30) et le purificateur de sang (20),
dans lequel l'unité de commande (80) ouvre la ligne de dérivation (70) au moyen de la troisième vanne de marche/arrêt (71) lorsqu'une pression mesurée par le deuxième capteur de pression (14) dépasse une valeur prédéterminée dans la sixième étape.

15. Dispositif de purification de sang (1) selon la revendication 14, comprenant en outre un troisième capteur de pression (62) destiné à mesurer une pression dans la ligne de retour de sang (60),
dans lequel l'unité de commande met fin au fonctionnement lorsqu'une pression mesurée par le troisième capteur de pression (62) dépasse une valeur prédéterminée dans la sixième étape.

16. Dispositif de purification de sang (1) selon la revendication 15, comprenant en outre :
une ligne de dérivation (70) pour contourner le purificateur de sang (20), et
une troisième vanne de marche/arrêt (71) pour ouvrir ou fermer la ligne de dérivation (70),
dans lequel l'unité de commande (80) ouvre la ligne de dérivation (70) au moyen de la troisième vanne de marche/arrêt (71) lorsqu'une différence entre la pression mesurée par le deuxième capteur de pression (14) et la pression mesurée par le troisième capteur de pression (62) dépasse une valeur prédéterminée dans la sixième étape.

17. Dispositif de purification de sang (1) selon l'une quelconque des revendications 1 à 16, dans lequel
la première transfusion est une solution saline physiologique, et
la seconde transfusion est différente de la solution saline physiologique.

18. Dispositif de purification du sang (1) selon la revendication 17, dans lequel la seconde transfusion contient un anticoagulant.
